# EUROPEAN PATENT SPECIFICATION

(11) **EP 1 867 335 B1**
(45) Date of publication and mention of the grant of the patent: **29.06.2011**
(21) Application number: 07117228.2
(22) Date of filing: 27.02.2006
(51) Int. Cl.: A61K 31/711, A61P 35/00, A61K 31/436

(54) **Oligodeoxyribonucleotides combined with rapamycin for treating cancer**
Oligonukleotide kombiniert mit Rapamycin zur Behandlung von Krebs
Des oligonucléotides combinees à de la rapamycine pour le traitement du cancer

(30) Priority: 03.03.2005 IT MI20050336; 28.10.2005 US 731404 P
(43) Date of publication of application: 19.12.2007
(62) Divisional of application: 06708536.5
(73) Proprietor: Gentium S.p.A., 22079 Villa Guardia (Como) (IT)
(72) Inventor: Iacobelli, Massimo, I-20152, MILANO (IT); Eissner, Gunter, 81667, MONACO (DE); Ferro, Laura Iris, I-20121, MILANO (IT)
(74) Representative: Pistolesi, Roberto

(56) References cited:
- EP-A- 0 558 833
- WO-A-98/48843
- WO-A-98/54313
- DE-A1- 19 740 384
- MITSIADES C S ET AL: "DEFIBROTIDE (DF) HAS ANTI-NEOPLASTIC ACTIVITY AGAINST MULTIPLE MYELOMA: CLINICAL IMPLICATIONS FOR THE INCORPORATION OF DF IN CYTOTOXIC CHEMOTHERAPEUTIC REGIMENS" BLOOD, W.B.SAUNDERS COMPANY, ORLANDO, FL, US, vol. 102, no. 11, 16 November 2003 (2003-11-16), page 693A, XP009041240 ISSN: 0006-4971
- MITSIADES C S ET AL: "DEFIBROTIDE (DF) TARGETS TUMOR-MICROENVIRONMENTAL INTERACTIONS AND SENSITIZES MULTIPLE MYELOMA AND SOLID TUMOR CELLS TO CYTOTOXIC CHEMOTHERAPEUTICS" BLOOD, W.B.SAUNDERS COMPANY, ORLANDO, FL, US, vol. 104, no. 11, PART 1, November 2004 (2004-11), page 85A, XP009067860 ISSN: 0006-4971
- MITA MONICA M ET AL: "Mammalian target of rapamycin: a new molecular target for breast cancer" CLINICAL BREAST CANCER, XX, XX, vol. 4, no. 2, June 2003 (2003-06), pages 126-137, XP008081078 ISSN: 1526-8209
- STEPHAN SUSANN ET AL: "Effect of rapamycin alone and in combination with antiangiogenesis therapy in an orthotopic model of human pancreatic cancer." CLINICAL CANCER RESEARCH : AN OFFICIAL JOURNAL OF THE AMERICAN ASSOCIATION FOR CANCER RESEARCH 15 OCT 2004, vol. 10, no. 20, 15 October 2004 (2004-10-15), pages 6993-7000, XP002465891 ISSN: 1078-0432
- STROEMBERG THOMAS ET AL: "Rapamycin sensitizes multiple myeloma cells to apoptosis induced by dexamethasone" BLOOD, vol. 103, no. 8, 15 April 2004 (2004-04-15), pages 3138-3147, XP002465892 ISSN: 0006-4971

## Description

### Formulations with angiogenesis-dependent tumour action

The subject of the present invention is a method for treating a tumor-affected mammalian by administering to said mammalian an effective amount of defibrotide in combination with rapamycin; in particular it relates to the use of defibrotide in combination with rapamycin for the treatment of angiogenesis-dependent tumors.

### Background of the invention

Angiogenesis is a multi-step process leading to the formation of new blood vessels from pre-existing vasculature and it is necessary for primary tumor growth, invasiveness and development of metastases (20). It is normally suppressed in the adult, where angiogenesis occurs transiently only during reproduction, development and wound healing. Beyond a critical volume, a tumor cannot expand further in the absence of neovascularization (12). To promote this, a tumor must acquire the angiogenic phenotype which is the result of the net balance between positive (pro-angiogenic) and negative (anti-angiogenic) regulators (16). However, tumors are highly heterogenous in vascular architecture, differentiation, and functional blood supply (24). These differences in size of avascular preangiogenic tumors may be due in part to the capacity of tumor cells to survive under differing degrees of hypoxia (18).
Evidence for the angiogenesis-dependency of certain tumors, such as multiple myeloma, even non-solid leukemias and lymphomas (8) and (21), as well as breast (25), colorectal (7), gastric (26), prostate (9), cervix (19), hepatocellular (23), and non-small cell lung cancer (13) came from the observation that the measure of the degree of angiogenesis, the microvessel density, is an independent prognostic factor for survival in the mentioned clinical entities (17). In a recent clinical study, again in breast carcinoma, it became clear that angiogenesis-related genes are important for clinical outcome, for example the vascular endothelial cell growth factor VEGF, the VEGF receptor FLT1, and metalloproteinase MMP9 (6).

### Definitions

The term defibrotide identifies a polydeoxyribonucleotide that is obtained by extraction from animal and/or vegetable tissues but which may also be be produced synthetically; the polydesoxyribonucleotide is normally used in the form of an alkalimetal salt, generally a sodium salt, and generally has a molecular weight of about 45-50 kDa (CAS Registry Number: 83712-60-1). Preferably, defibrotide presents the physical/chemical characteristics described in (4) and (5), which are incorporated herein by reference.

### DESCRIPTION OF THE INVENTION

We have recently developed a model for an alternative pathway of tumor angiogenesis. In addition to the endothelial cell sprouting from pre-existing vessels, we suggest that blood borne endothelial cells might also give rise to the tumor vasculature. These endothelial-like cells (ELC) can transdifferentiate from tumor-associated dendritic cells under specific culture conditions (11). Briefly, monocytes are elutriated from leukapheresis products of healthy human blood donors and cultured in the presence of granulocyte-macrophage-colony stimulating factor (GM-CSF) and interleukin 4 (IL-4) to stimulate the differentiation of dendritic cells (DC). In addition, cells are treated with a cocktail specifically released by tumor cells (M-CSF, IL.6 and lactate, Gottfried et al., manucript submitted) to promote the outgrowth of tumor-associated dendritic cells (TuDC).

These TuDC-ELC acquire the phenotype of endothelial cells (FactorVIII related Ag, vWF) while they lose monocytic (CD14) and dendritic cell markers (CD1a). Importantly, they do not express CD34, nor CD133 or CD146 which proves that they are real transdifferentiation products and no contaminants of either circulating endothelial progenitors (CD34, CD133) or mature circulating endothelial cells (CD146). In addition, they are able to form tube-like structures in MatrigelTM, an in vitro assay of angiogenesis.

The MatrigelTM assay is one of the most popular and widely used in vitro angiogenesis assays (22).

MatrigelTM is a semisolid synthetic mixture of extracellular matrix proteins which simulate the matrix that physiologically exist beneath the endothelial cell wall of a blood vessel. When the cells of question are seeded onto this matrix in microscopic chamber slides, they are activated to form tubular structures in 3-7 days, but only in the case that they have an endothelial phenotype. Therefore, this assay is suitable to show the potential capacity of cells to give rise to a tumor vasculature.

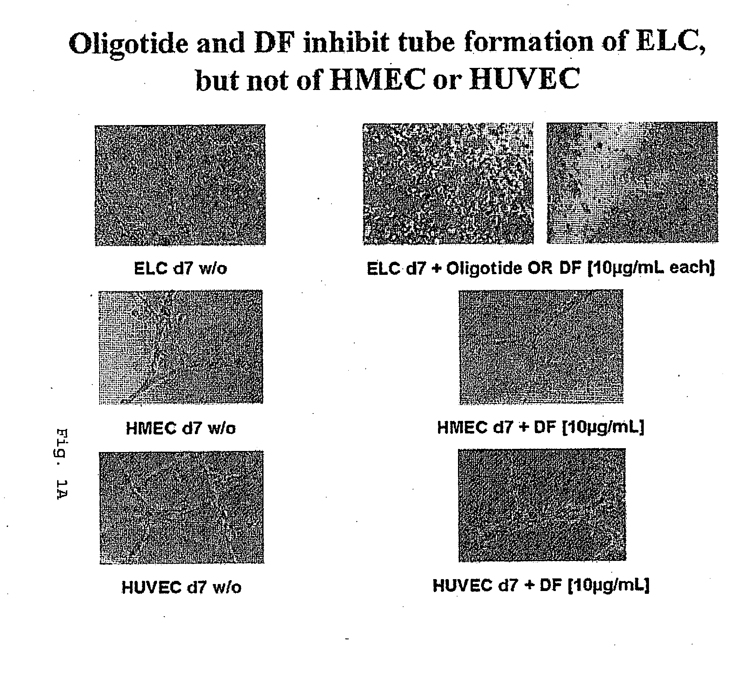

Our data data demonstrate that defibrotide in clinical and subclinical concentrations can inhibit tube formation of transdifferentiating ELC (TuDC-ELC) in MatrigelTM. TuDC-ELC and mature, differentiated endothelial cells, [human umbilical vene (HUVEC) or microvascular endothelial cells (HMEC) as "stable" controls] were incubated in the presence or absence of Defibrotide (10µg/mL each) for 7 days. Importantly, after a single addition of Defibrotide, HUVEC and HMEC are not affected in their tube formation potential, suggesting that Defibrotide only target transdifferentiating endothelial cells (Figure 1 A). However, when Defibrotide was added repeatedly, it could also block angiogenesis of mature, fully differentiated endothelial cells (see below).

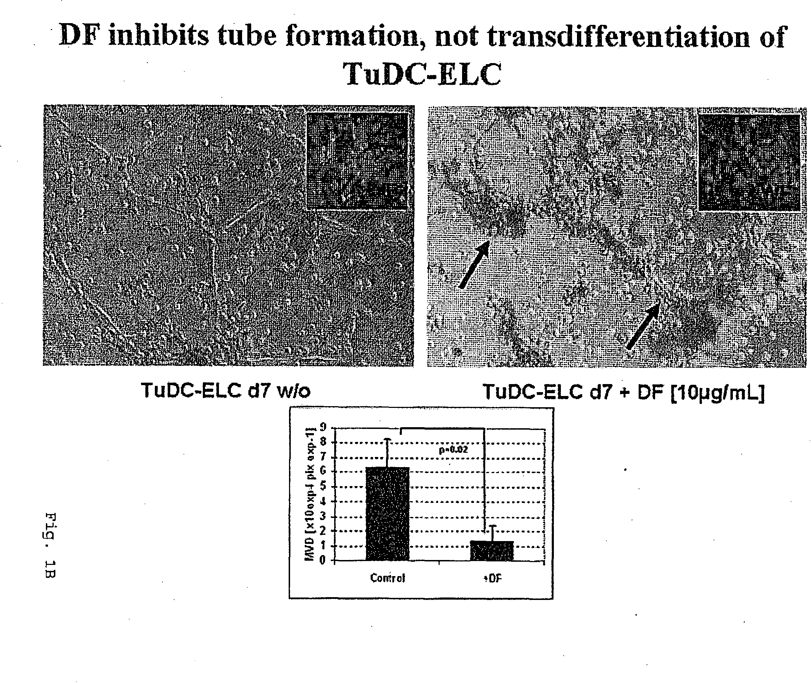

By the help of a complimentary software from the NIH (Image J, http://rsb.info.nih.gov/ij/), we are able to quantify these effects, the total length of tubes and the area of the photograph are assessed, the microvascular density (MVD) is then given in total length/area [pix-1]. DF significantly (p=0.02, TTEST) downregulates MVD of TuDC-ELC (Figure 1 B).

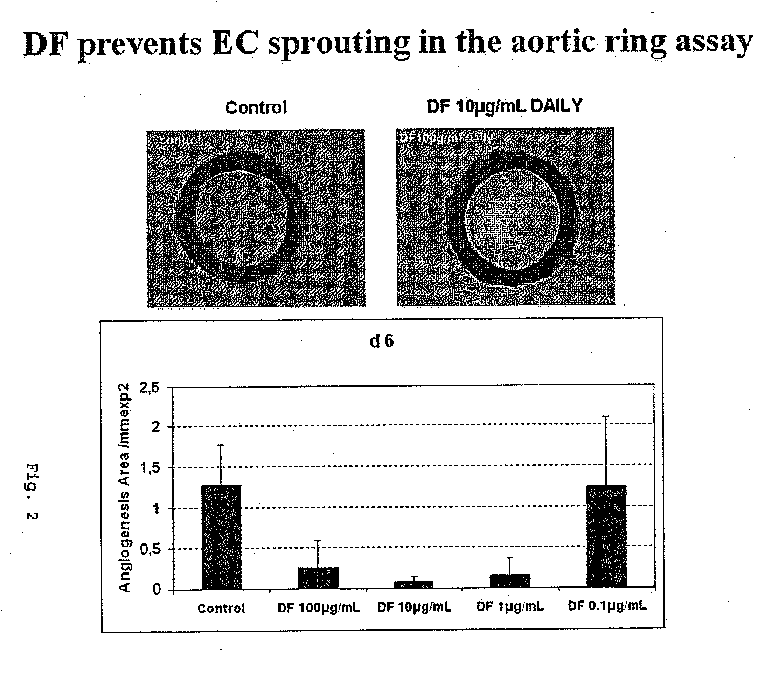

To support these data with an alternative angiogenesis assay the sprouting of rat aorta endothelial cells in MatrigelTM was prevented by nearly 100%, when DF was applied on a daily basis (Figure 2), suggesting that DF not only acts on transdifferentiating, but also on mature, fully differentiated endothelial cells.

The aortic ring assay investigates macrovascular endothelial cells. But often, the tumor vasculature consists of microvascular endothelial cells. Therefore, a third in vitro angiogenesis assay was performed on the basis of microvascular endothelial cells vascularizing through a layer of dermal fibroblasts after 9-11 days of culture. These vessel-like structures can subsequently be visualized by staining for CD31 and vWF.

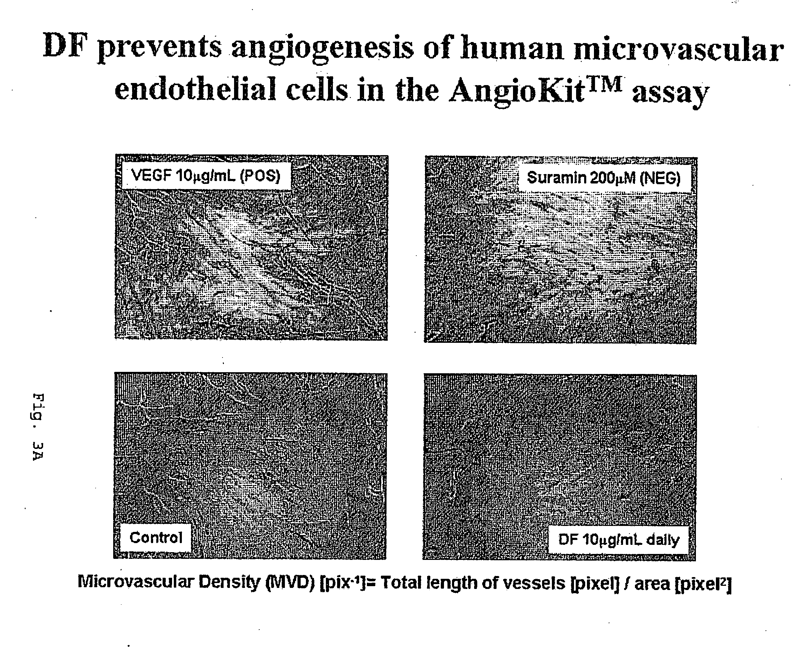

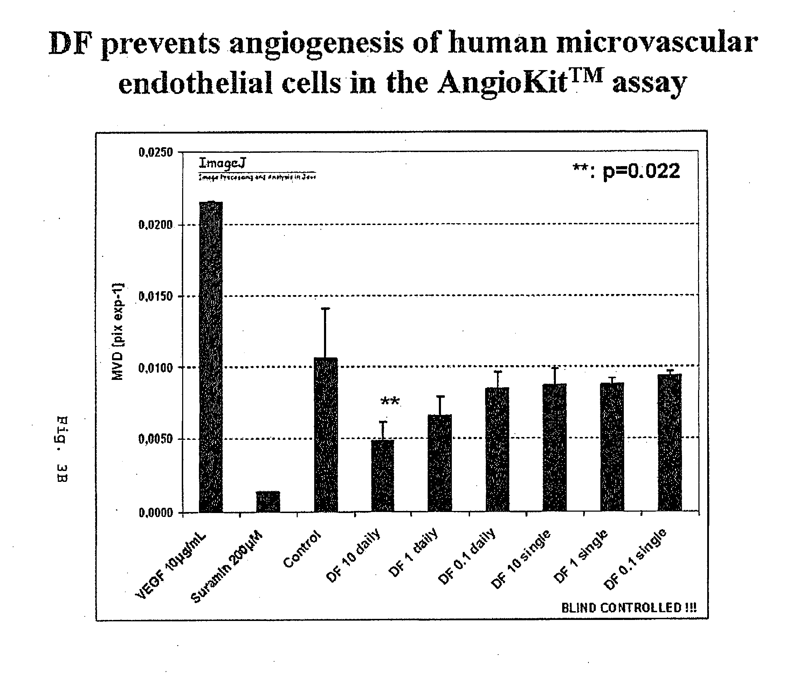

As demonstrated in Figure 3 (A and B), DF can also block angiogenesis of human microvascular endothelial cells with a superiority for the daily application. Interestingly, concentrations around 10 µg/mL appear to be the most effective. A single application of DF could not significantly block angiogenesis.

Taken together, our data strongly suggest that defibrotide can block angiogenesis of tumor-associated transdifferentiating endothelial cells and those that arise from already existing vascular cells.

It is subject to ongoing studies whether defibrotide also inhibit angiogenesis in vivo. We are currently performing a dorsal skin chamber assay (14) that investigates the effect of defibrotide in a highly vascularized human gastric carcinoma mouse model (Xenograft system). First data clearly show that the microvascular density (MVD) of DF-treated tumors is lower than that of control tumors. This set of experiments will be reproduced in due time.

The mechanism of action by which DF can block angiogenesis remains to be elucidated, but preliminary evidence from Western Blot analyses suggest a downregulating effect of DF on activated p70S6 kinase (p-p70S6), a mitogen-activated protein kinase.

Additional evidence for the impact of p70S6 kinase was obtained from another tube formation assay with HMEC incubated in the presence or absence of the p70S6 kinase inhibtor DRB.

There are also first clinical data available for patients (pts.) having received allogeneic stem cell transplantation (SCT): In a cohort of 17 defibrotide-treated pts a striking decline in serum VEGF levels has been seen, also suggesting that defibrotide might act through growth factor withdrawal for sprouting tumor endothelial cells.

Defibrotide is a strong candidate for a therapy of angiogenesis-dependent tumors and is used in combination with rapamycin (14).

Interestingly, rapamycin has the negative side effect of pro-thrombotic activity (15) that could be attenuated by the simultaneous application of the antithrombotic and fibrionolytic defibrotide.

### References

1. US5646127
2. US5646268
3. US6046172
4. US4985552
5. US5223609
6. 't Veer,L.J., et al.(2002) Gene expression profiling predicts clinical outcome of breast cancer. Nature, 415, 530-536.
7. Abdalla,S.A., et al.(1999) Prognostic relevance of microvessel density in colorectal tumours. Oncol.Rep., 6, 839-842.
8. Andersen,N.F., et al. (2005) Syndecan-1 and angiogenic cytokines in multiple myeloma: correlation with bone marrow angiogenesis and survival. Br.J.Haematol., 128, 210-217.
9. Bostwick,D.G. & Iczkowski, K.A. (1998) Microvessel density in prostate cancer: prognostic and therapeutic utility. Semin.Urol.Oncol., 16, 118-123.
10. Eissner,G., et al. (2002) Fludarabine induces apoptosis, activation, and allogenicity in human endothelial and epithelial cells: protective effect of defibrotide. Blood, 100, 334-340.
11. Fernandez,P.B., et al. (2001) Dendritic cells derived from peripheral monocytes express endothelial markers and in the presence of angiogenic growth factors differentiate into endothelial-like cells. Eur.J.Cell Biol., 80, 99-110.
12. Folkman,J., et al. (1971) Isolation of a tumor factor responsible for angiogenesis. J.Exp.Med., 133, 275-288.
13. Fontanini,G., et al. (1995) Microvessel count predicts metastatic disease and survival in non-small cell lung cancer. J.Pathol., 177, 57-63.
14. Guba,M., et al.(2002) Rapamycin inhibits primary and metastatic tumor growth by antiangiogenesis: involvement of vascular endothelial growth factor. Nat.Med., 8, 128-135.
15. Guba,M., et al. (2005) Rapamycin induces tumor-specific thrombosis via tissue factor in the presence of VEGF. Blood.
16. Hanahan,D. & Folkman,J. (1996) Patterns and emerging mechanisms of the angiogenic switch during tumorigenesis. Cell, 86, 353-364.
17. Hasan,J., et al. (2002) Intra-tumoural microvessel density in human solid tumours. Br.J.Cancer, 86, 1566-1577.
18. Helmlinger,G., et al. (1997) Interstitial pH and pO2 gradients in solid tumors in vivo: high-resolution measurements reveal a lack of correlation. Nat.Med., 3, 177-182.
19. Kainz,C., et al. (1995) Prognostic value of tumour microvessel density in cancer of the uterine cervix stage IB to IIB. Anticancer Res., 15, 1549-1551.
20. Morabito,A., et al. (2004) Antiangiogenic strategies, compounds, and early clinical results in breast cancer. Crit Rev.Oncol.Hematol., 49, 91-107.
21. Podar,K. & Anderson, K.C. (2005) The pathophysiologic role of VEGF in hematologic malignancies: therapeutic implications. Blood, 105, 1383-1395.
22. Staton, C.A., et al. (2004) Current methods for assaying angiogenesis in vitro and in vivo. Int.J.Exp.Pathol., 85, 233-248.
23. Sun,H.C., et al. (1999) Microvessel density of hepatocellular carcinoma: its relationship with prognosis. J.Cancer Res.Clin.Oncol., 125, 419-426.
24. Verheul,H.M., et al. (2004) Are tumours angiogenesis-dependent? J.Pathol., 202, 5-13.
25. Weidner,N., et al. (1992) Tumor angiogenesis: a new significant and independent prognostic indicator in early-stage breast carcinoma. J.Natl.Cancer Inst., 84, 1875-1887.
26. Xiangming,C., et al. (1998) Angiogenesis as an unfavorable factor related to lymph node metastasis in early gastric cancer. Ann.Surg.Oncol., 5, 585-589.

## Claims

1. Use of defibrotide in combination with rapamicyn for the manufacture of a pharmaceutical formulation for the treatment of angiogenesis-dependent tumour.

2. Use according to claim 1, **characterized in that** said defibrotide is obtained by extraction from animal and/or vegetable tissues, preferably from mammalian organs.

3. Use according to claim 1, **characterized in that** said defibrotide is obtained synthetically.

4. Use according to claim 1, **characterized in that** said angiogenesis-dependent tumor is multiple myeloma.

5. Use according to claim 1, **characterized in that** said angiogenesis-dependent tumor is breast carcinoma.

6. Use according to claim 1, **characterized in that** said formulation is administered to a mammalian.

7. Use according to claim 1, **characterized in that** said formulation is administered to a human.

8. Use according to claim 1, **characterized in that** said formulation is administered intravenously.

9. Use according to claim 1, **characterized in that** said formulation is an aqueous solution.

10. Use according to claim 1, **characterized in that** said formulation contains customary excipients and/or adjuvants.

## Patentansprüche

1. Verwendung von Defibrotid in Kombination mit Rapamycin zur Herstellung einer pharmazeutischen Formulierung zur Behandlung von Angiogenese-abhängigen Tumoren.

2. Verwendung nach Anspruch 1, **dadurch gekennzeichnet, dass** das genannte Defibrotid durch Extraktion von tierischen und/oder pflanzlichen Geweben, vorzugsweise von Säugetierorganen, gewonnen wird.

3. Verwendung nach Anspruch 1, **dadurch gekennzeichnet, dass** das genannte Defibrotid synthetisch hergestellt wird.

4. Verwendung nach Anspruch 1, **dadurch gekennzeichnet, dass** der genannte Angiogenese-abhängige Tumor ein multiples Myelom ist.

5. Verwendung nach Anspruch 1, **dadurch gekennzeichnet, dass** der genannte Angiogenese-abhängige Tumor ein Brustkarzinom ist.

6. Verwendung nach Anspruch 1, **dadurch gekennzeichnet, dass** die Formulierung einem Säugetier verabreicht wird.

7. Verwendung nach Anspruch 1, **dadurch gekennzeichnet, dass** die Formulierung einem Menschen verabreicht wird.

8. Verwendung nach Anspruch 1, **dadurch gekennzeichnet, dass** die Formulierung intravenös verabreicht wird.

9. Verwendung nach Anspruch 1, **dadurch gekennzeichnet, dass** die Formulierung eine wässrige Lösung ist.

10. Verwendung nach Anspruch 1, **dadurch gekennzeichnet, dass** die Formulierung herkömmliche Exzipienten und/oder Adjuvantien enthält.

## Revendications

1. Utilisation de défibrotide en combinaison avec la rapamycine pour la fabrication d'une formulation pharmaceutique destinée au traitement d'une tumeur dépendante de l'angiogenèse.

2. Utilisation selon la revendication 1, **caractérisée en ce que** ledit defibrotide est obtenu par une extraction à partir de tissus animaux et/ou végétaux, de préférence à partir d'organes de mammifères.

3. Utilisation selon la revendication 1, **caractérisée en ce que** ledit défibrotide est obtenu de manière synthétique.

4. Utilisation selon la revendication 1, **caractérisée en ce que** ladite tumeur dépendante de l'angiogenèse est un myélome multiple.

5. Utilisation selon la revendication 1, **caractérisée en ce que** ladite tumeur dépendante de l'angiogenèse est un carcinome mammaire.

6. Utilisation selon la revendication 1, **caractérisée en ce que** ladite formulation est administrée à un mammifère.

7. Utilisation selon la revendication 1, **caractérisée en ce que** ladite formulation est administrée à un humain.

8. Utilisation selon la revendication 1, **caractérisée en ce que** ladite formulation est administrée par voie intraveineuse.

9. Utilisation selon la revendication 1, **caractérisée en ce que** ladite formulation est une solution aqueuse.

10. Utilisation selon la revendication 1, **caractérisée en ce que** ladite formulation contient des excipients et/ou adjuvants habituels.
